# EUROPEAN PATENT APPLICATION

(11) **EP 4 461 731 A1**
(43) Date of publication of application: **13.11.2024**
(21) Application number: 23172992.2
(22) Date of filing: 12.05.2023
(51) Int. Cl.: C07D 473/34, C07D 473/40, C07D 487/04, A61K 31/52, A61P 35/00

(54) **LIGANDS OF THE M6A-RNA READERS**

(71) Applicant: Universität Zürich, 8006 Zürich (CH)
(72) Inventor: ZALESAK, Frantisek, 8006 Zürich (CH); NAI, Francesco, 8006 Zürich (CH); BEDI, Rajiv, 8006 Zürich (CH); HEROK, Marcin, 8006 Zürich (CH); ERRANI, Francesco, 8006 Zürich (CH); IVERNIZZI, Annalisa, 8006 Zürich (CH); LI, Yaozong, 8006 Zürich (CH); CAFLISCH, Amedeo, 8006 Zürich (CH)
(74) Representative: Schulz Junghans Patentanwälte PartGmbB

(57) **Abstract**

The present invention relates to a compound of formula (I) or (II) 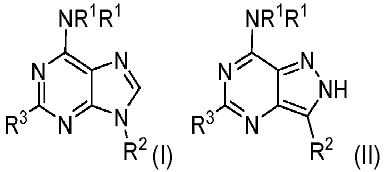 for use as a medicament in the treatment of cancer.

## Description

### Field

The present invention relates to a compound for use as a ligand of m6a-RNA readers.

### Background

N6-Methyladenosine (m6A) is an abundant modification in mRNA and DNA. It is found within some viruses, and most eukaryotes including mammals, insects, plants and yeast. It is also found in tRNA, rRNA, and small nuclear RNA (snRNA) as well as several long non-coding RNA, such as Xist. The biological functions of m6A are mediated through a group of RNA binding proteins that specifically recognize the methylated adenosine on RNA. These binding proteins are named m6A readers. The YT521-B homology (YTH) domain family of proteins (YTHDF1, YTHDF2, YTHDF3 and YTHDC1) have been characterized as direct m6A readers and have a conserved m6A-binding pocket. YTH domain-containing protein 1 is a protein that in humans is encoded by the YTHDC1 gene. YTHDC1 is a nuclear protein involved in splice site selection that localises to YT bodies; dynamic subnuclear compartments, which first appear at the beginning of S-phase in the cell cycle and disperse during mitosis. Alternative splicing, however is known to be altered in a number of diseases and is particularly relevant to cancer. YTHDC1 has been shown to splice mRNA transcripts which have oncological importance, regulating tumour functions such as hypoxia associated vascular endothelial growth factor (VEGF), DNA damage associated breast cancer 1 (BRCA1) and hormonal growth driver; the progesterone receptor (PGR). It has further been shown that YTHDC1 is overexpressed in acute myeloid leukemia (AML) and that it is required for the proliferation and survival of AML cells, while knockout of YTHDC1 blocks AML development and maintenance as well as self-renewal of leukemia stem cells (LSCs) in vivo.

However, there are no potent YTHDC1 inhibitors known, much less with sufficient efficacy for the treatment of cancer.

Based on the above-mentioned state of the art, the objective of the present invention is to provide novel ligands of m6A-RNA readers, particularly of YTDC1. This objective is attained by the subject-matter of the independent claims of the present specification, with further advantageous embodiments described in the dependent claims, examples, figures and general description of this specification.

### Summary of the Invention

A first aspect of the invention relates to a compound of formula (I) or (II), particularly of (I) 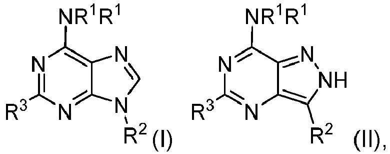 wherein
R¹ is independently selected from -H and -C₁-C₃-alkyl, particularly -H and -Me or cyclopropyl, more particularly -H and -Me, wherein at least one of R¹ is H,
R³ is selected from -Br, -Cl, -CN, -F and -NO₂, particularly -Cl,
R² is selected from -C₁-C₃-alkyl and a moiety of formula (III) or (IV), particularly R² is a moiety of formula (III) or (IV),
   - 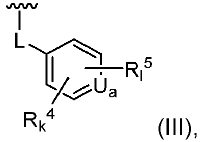 wherein
      L of formula (I) is a linker comprising -(CH₂)ₙ- or -(CH₂)ₙ-Y- and
      L of formula (II) is a linker comprising -(CH₂)ₙ- or -Y-(CH₂)ₙ-, wherein Y is selected from -O-, -S-, -SO₂-, -NH- and -C(=O)-NH-, and
      n is 0 to 3, k is 0 to 3, particularly 0 to 2, more particularly 1 or 2, I is 0 or 1,
      U is CH or a heteroatom, particularly CH or N, more particularly CH,
      a is 0 or 1, particularly 1,
      R⁴ is independently selected from
         - -Cl, -Br, -CH₂F, -CHF₂, -CF₃ and
         - -C(=O)O-C₁-C₃-alkyl, -C(=O)OH, -O-C₁-C₃-alkyl, - C(=O)-NR⁶R⁶, -C₁-C₃-alkyl-OH, and
         - -NR⁶R⁶,
         wherein R⁶ is independently selected from
         - -H, -C(=O)-O-tert-butyl, -C₁-C₃-alkyl, and - C(=O)-CF₃, particularly from -H and Me,
      R⁵ is selected from
         - -NH-SO₂-C₁-C₃-alkyl, -NH-SO₂-(CH₂)ₛ-OH and
         - -NH-SO₂-(CH₂)ₛ-R⁷, wherein R⁷ is selected from an aryl and a heterocycle, wherein the heterocycle is a five or six-membered heterocycle and wherein the heterocycle is an aliphatic heterocycle or an aromatic heterocycle, and
         - -(CH₂)ₛ-R⁸, wherein R⁸ is a four or five membered ring,
         s is 0 to 3, or
   - 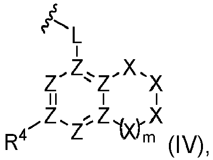 wherein
      L of formula (I) is a linker comprising -(CH₂)ₙ- or -(CH₂)ₙ-Y- and
      L of formula (II) is a linker comprising -(CH₂)ₙ- or -Y-(CH₂)ₙ-, wherein Y is selected from -O-, -S-, -SO₂-, -NH- and -C(=O)-NH-, and
      n is 0 to 3, m is 0 or 1,
      each X and Z are selected from C, NR³, SO₂ and O, wherein R³ is -H or -C₁-C₃-alkyl-NH₂ and wherein Z is particularly C,
      R⁴ is defined as above,
with the exception of

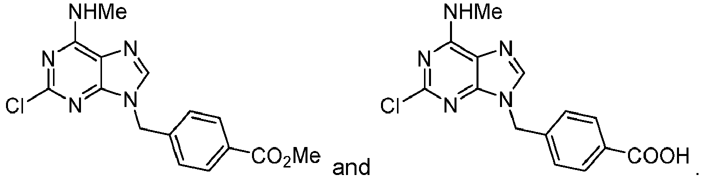

A second aspect of the invention relates to a compound selected from any one of (V) to (XI) for use as a medicament

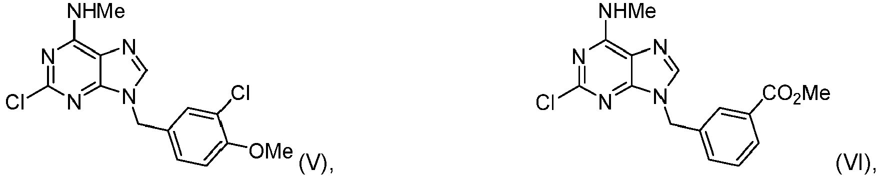

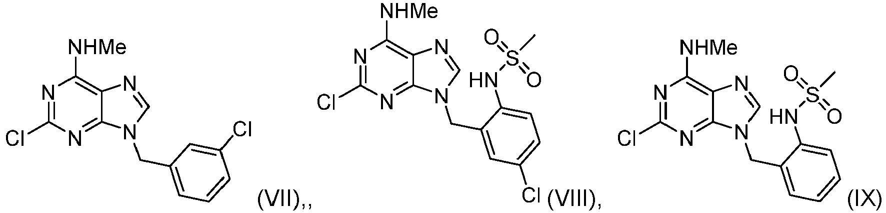

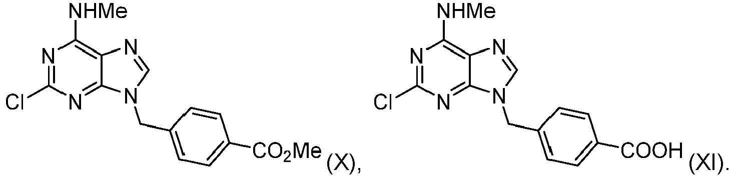

A third aspect of the invention relates to a compound according to the first aspect of the invention for use in the treatment of a disease, wherein the disease is cancer.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *TR-FRET* in the context of the present specification relates to time-resolved fluorescence energy transfer.

The term TSA in the context of the present specification relates to thermal shift assay which measures the thermal denaturation temperature and hence stability of a protein under varying conditions such as variations in drug concentration, buffer pH, ionic strength, redox potential or sequence mutation. The thermal denaturation temperature is measured via differential scanning fluorimetry (DSF) using specialised fluorogenic dyes. The binding of low molecular weight ligands can increase the thermal stability of a protein.

The term *IC₅₀* in the context of the present specification relates to the half maximal inhibitory concentration which states the potency if a substance in inhibiting a specific biochemical function. IC₅₀ is a quantitative measure that indicates the concentration of a particular inhibitor required to inhibit a biological process or biological component by 50%.

The term *Gl₅₀* in the context of the present specification relates to the half maximal growth inhibition. GI₅₀ is a quantitative measure that indicates the concentration of a particular inhibitor required to inhibit the cell proliferation by 50%.

The term in the context of the present specification relates to

Any patent document cited herein shall be deemed incorporated by reference herein in its entirety.

The term *alkyl* in the context of the present specification relates to a saturated linear or unsaturated linear or branched hydrocarbon.

The term C₁-C₃ *alkyl* in the context of the present specification relates to a saturated linear or branched hydrocarbon having 1, 2 or 3 carbon atoms. Non-limiting examples for a C₁-C₃ alkyl are methyl, ethyl, propyl, prop-2-enyl, cyclo-propyl. In certain embodiments, a C₁-C₃ alkyl is a methyl, ethyl or propyl moiety.

Where used in the context of chemical formulae, the following abbreviations may be used: Me is methyl CH₃, *Et* is ethyl -CH₂CH₃, *Prop* is propyl -(CH₂)₂CH₃ (n-propyl, n-pr) or -CH(CH₃)₂ (iso-propyl, i-pr), *but* is butyl -C₄H₉, -(CH₂)₃CH₃, -CHCH₃CH₂CH₃, -CH₂CH(CH₃)₂ or -C(CH₃)₃.

In the context of the present specification, the term alkyl also comprises unsaturated hydrocarbons like alkene and alkyne.

The term *alkene* in the context of the present specification relates to a hydrocarbon comprising a double bond. Unsubstituted alkene is of formula -CH=CH- when being located intramolecularly, and of formula -CH-CH₂ when being a terminal moiety. An unsubstituted alkene consists of C and H only. A substituted alkene may comprise substituents as defined herein for substituted alkyl.

The term *alkyne* in the context of the present specification relates to a hydrocarbon comprising a triple bond. Unsubstituted alkyne is of formula -C≡C- when being located intramolecularly, and of formula -C≡CH (-C₂H) when being a terminal moiety. An unsubstituted alkyne consists of C and H only. A substituted alkyne may comprise substituents as defined herein for substituted alkyl.

The term *aryl* in the context of the present specification relates to a cyclic aromatic C₅-C₁₀ hydrocarbon. Examples of aryl include, without being restricted to, phenyl and naphthyl.

The term *heterocycle* in the context of the present specification relates to a cyclic aromatic or aliphatic C₃-C₉ hydrocarbon that comprises at least one heteroatom (e.g. N, O, S). Examples for heteroaryl include, without being restricted to, pyrrole, thiophene, furan, imidazole, pyrazole, thiazole, oxazole, pyridine, pyrimidine, thiazin, quinoline, benzofuran and indole.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

The term *cancer* as used in the context of the present specification relates to malignant neoplastic disease; the terms "cancer" and "malignant neoplastic disease" are used synonymously herein. They specifically include carcinoma (epithelial derived cancer), sarcoma (connective tissue derived cancer), lymphoma and leukemia, germ-cell derived tumours and blastomas. Particular alternatives of any of the aspects and embodiments disclosed herein are directed at the use of the compounds and compositions of the invention in treatment of solid tumours. Other alternatives of any of the aspects and embodiments disclosed herein are directed at the use of the combinations of the invention in treatment of cancers such as renal cancer, breast cancer, acute myeloid leukemia, hepatocellular carcinoma, and lung adenocarcinoma.

The term *inhibitor* in the context of the present specification relates to any pharmaceutically acceptable agent or compound that may be used to interact with and specifically interfere with the biological activity of its designated target, in case of the present invention YTH protein domain, particularly ZTH domain-containing protein 1 (YTHDC1).

As used herein, the term *treating* or *treatment* of any disease or disorder (e.g. cancer) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Detailed Description of the Invention

A first aspect of the invention relates to a compound of formula (I) or (II), particularly of (I) 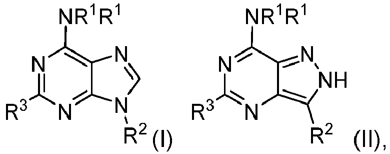 wherein
R¹ is independently selected from -H and -C₁-C₃-alkyl, particularly -H and -Me or cyclopropyl, more particularly -H and -Me, wherein at least one of R¹ is H,
R³ is selected from -Br, -Cl, -CN, -F and -NO₂, particularly -Cl,
R² is selected from -C₁-C₃-alkyl and a moiety of formula (III) or (IV), particularly R² is a moiety of formula (III) or (IV), 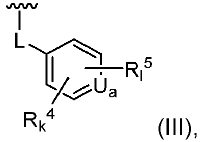 wherein
   L of formula (I) is a linker comprising -(CH₂)ₙ- or -(CH₂)ₙ-Y- and
   L of formula (II) is a linker comprising -(CH₂)ₙ- or -Y-(CH₂)ₙ-, wherein Y is selected from -O-, -S-, -SO₂-, -NH- and -C(=O)-NH-, and
   n is 0 to 3, k is 0 to 3, particularly 0 to 2, more particularly 1 or 2,
   I is 0 or 1,
   U is CH or a heteroatom, particularly CH or N, more particularly CH,
   a is 0 or 1, particularly 1,
   R⁴ is independently selected from
      - -Cl, -Br, -CH₂F, -CHF₂, -CF₃ and
      - -C(=O)O-C₁-C₃-alkyl, -C(=O)OH, -O-C₁-C₃-alkyl, - C(=O)-NR⁶R⁶, -C₁-C₃-alkyl-OH, and
      - -NR⁶R⁶,
      wherein R⁶ is independently selected from
      - -H, -C(=O)-O-tert-butyl, -C₁-C₃-alkyl, and - C(=O)-CF₃, particularly from -H and Me,
   R⁵ is selected from
      - -NH-SO₂-C₁-C₃-alkyl, -NH-SO₂-(CH₂)ₛ-OH and
      - -NH-SO₂-(CH₂)ₛ-R⁷, wherein R⁷ is selected from an aryl and a heterocycle, wherein the heterocycle is a five or six-membered heterocycle and wherein the heterocycle is an aliphatic heterocycle or an aromatic heterocycle, and
      - -(CH₂)ₛ-R⁸, wherein R⁸ is a four or five membered ring,
      s is 0 to 3, or

   - 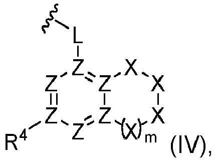 wherein
      L of formula (I) is a linker comprising -(CH₂)ₙ- or -(CH₂)ₙ-Y- and
      L of formula (II) is a linker comprising -(CH₂)ₙ- or -Y-(CH₂)ₙ-, wherein Y is selected from -O-, -S-, -SO₂-, -NH- and -C(=O)-NH-, and
      n is 0 to 3, m is 0 or 1,
      each X and Z are selected from C, NR³, SO₂ and O, wherein R³ is -H or -C₁-C₃-alkyl-NH₂ and wherein Z is particularly C,
      R⁴ is defined as above,
with the exception of

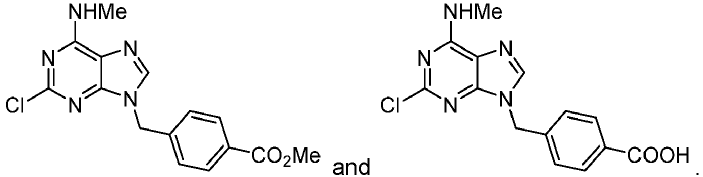

Formula (II) includes all resonance structures of said formula, e.g.

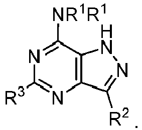

In certain embodiments, the compound is a compound of formula (I) 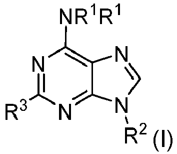 wherein
R¹ is independently selected from -H and -C₁-C₃-alkyl, particularly -H and -Me or cyclopropyl, more particularly -H and -Me, wherein at least one of R¹ is H,
R³ is selected from -Br, -Cl, -CN, -F and -NO₂, particularly -Cl,
R² is selected from -C₁-C₃-alkyl and a moiety of formula (III) or (IV), particularly R² is a moiety of formula (III) or (IV),
   - 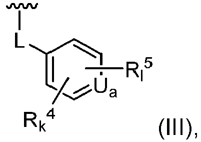 wherein
      L is a linker comprising -(CH₂)ₙ- or -(CH₂)ₙ-Y- wherein Y is selected from -O-, -S-, -SO₂-, -NH- and -C(=O)-NH-, and
      n is 0 to 3, k is 0 to 3, particularly 0 to 2, more particularly 1 or 2,
      I is 0 or 1,
      U is CH or a heteroatom, particularly CH or N, more particularly CH,
      a is 0 or 1, particularly 1,
      R⁴ is independently selected from
         - -Cl, -Br, -CH₂F, -CHF₂, -CF₃ and
         - -C(=O)O-C₁-C₃-alkyl, -C(=O)OH, -O-C₁-C₃-alkyl, - C(=O)-NR⁶R⁶, -C₁-C₃-alkyl-OH, and
         - -NR⁶R⁶,
         wherein R⁶ is independently selected from
         - -H, -C(=O)-O-tert-butyl, -C₁-C₃-alkyl, and - C(=O)-CF₃, particularly from -H and Me,
      R⁵ is selected from
         - -NH-SO₂-C₁-C₃-alkyl, -NH-SO₂-(CH₂)ₛ-OH and
         - -NH-SO₂-(CH₂)ₛ-R⁷, wherein R⁷ is selected from an aryl and a heterocycle, wherein the heterocycle is a five or six-membered heterocycle and wherein the heterocycle is an aliphatic heterocycle or an aromatic heterocycle, and
         - -(CH₂)ₛ-R⁸, wherein R⁸ is a four or five membered ring,
         s is 0 to 3,
         or
   - 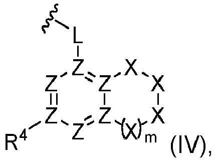 wherein
      L is a linker comprising -(CH₂)ₙ- or -(CH₂)ₙ-Y- and
      wherein Y is selected from -O-, -S-, -SO₂-, -NH- and -C(=O)-NH-, and n is 0 to 3, m is 0 or 1,
      each X and Z are selected from C, NR³, SO₂ and O, wherein R³ is -H or -C₁-C₃-alkyl-NH₂ and wherein Z is particularly C,
      R⁴ is defined as above,
with the exception of

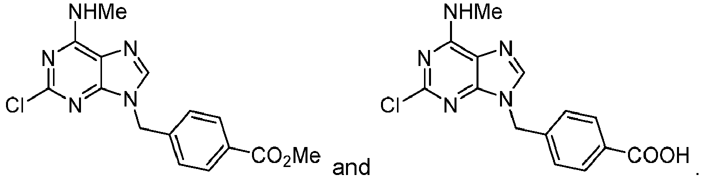

In certain embodiments, R² is a moiety of formula (III) or (IV).

In certain embodiments the compound is a compound of formula (Ia) or (IIa), particularly of (Ia) 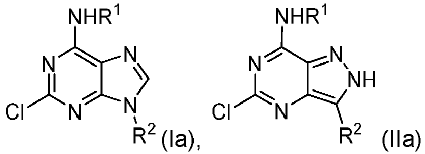 wherein
R¹ is selected from -H and -C₁-C₃-alkyl, particularly -H and -Me or cyclopropyl, more particularly -Me,
R² is selected from -C₁-C₃-alkyl and a moiety of formula (Illa) or (lVa), particularly R² is a moiety of formula (IIIa) or (IVa),
   - 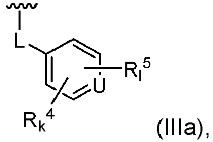 wherein
      L of formula (I) is a linker comprising -(CH₂)ₙ- or -(CH₂)ₙ-Y- and
      L of formula (II) is a linker comprising -(CH₂)ₙ- or -Y-(CH₂)ₙ-, wherein Y is selected from -O-, -S-, -SO₂-, -NH- and -C(=O)-NH-, and
      and n is 0 to 3,
      k is 0 to 3, particularly 0 to 2, more particularly 1 or 2,
      I is 0 or 1,
      U is CH or a heteroatom, particularly CH or N, more particularly CH,
      R⁴ is selected from
         - -Cl, -Br, -CH₂F, -CHF₂, -CF₃, and
         - -C(=O)O-C₁-C₃-alkyl, -C(=O)OH, -O-C₁-C₃-alkyl, - C(=O)-N R⁶R⁶, -C₁-C₃-alkyl-OH, and
         - -NR⁶R⁶,
         wherein R⁶ is independently selected from
         - -H, -C(=O)-O-tert-butyl, -C₁-C₃-alkyl and - C(=O)-CF₃, particularly from -H and Me,
      R⁵ is selected from
         - -NH-SO₂-C₁-C₃-alkyl, -NH-SO₂-(CH₂)ₛ-OH, and
         - -NH-SO₂-(CH₂)ₛ-R⁷,
            wherein R⁷ is selected from an aryl or a heterocycle,
            wherein the heterocycle is a five or six-membered heterocycle and wherein the heterocycle is an aliphatic heterocycle or an aromatic heterocycle, and
         - -(CH₂)ₛ-R⁸,
            wherein R⁸ is a four or five membered ring,
         s is 0 to 3,
   - 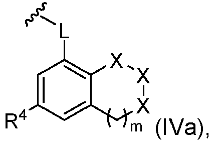 wherein
      L of formula (I) is a linker comprising -(CH₂)ₙ- or -(CH₂)ₙ-Y- and
      L of formula (II) is a linker comprising -(CH₂)ₙ- or -Y-(CH₂)ₙ-, wherein Y is selected from -O-, -S-, -SO₂-, -NH- and -C(=O)-NH-, and
      n is 0 to 3,
      n is 0 or 1,
      m is 0 or 1,
      X is independently selected from CH, NR³, SO₂ and O, wherein
      R³ is selected from -H and -C₁-C₃-alkyl-NH₂,
R⁴ is defined as above.

Formula (Ila) includes all resonance structures of said formula, e.g.

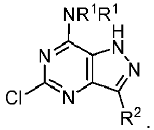

In certain embodiments the compound is a compound of formula (Ia) 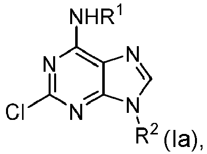 wherein
R¹ is selected from -H and -C₁-C₃-alkyl, particularly -H and -Me or cyclopropyl, more particularly -Me,
R² is selected from -C₁-C₃-alkyl and a moiety of formula (Illa) or (lVa), particularly R² is a moiety of formula (IIIa) or (IVa),
   - 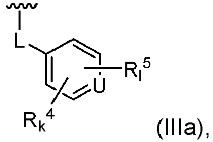 wherein
      L is a linker comprising -(CH₂)ₙ- or -(CH₂)ₙ-Y- and
      wherein Y is selected from -O-, -S-, -SO₂-, -NH- and -C(=O)-NH-, and
      and n is 0 to 3,
      k is 0 to 3, particularly 0 to 2, more particularly 1 or 2,
      I is 0 or 1,
      U is CH or a heteroatom, particularly CH or N, more particularly CH,
      R⁴ is selected from
         - -Cl, -Br, -CH₂F, -CHF₂, -CF₃, and
         - -C(=O)O-C₁-C₃-alkyl, -C(=O)OH, -O-C₁-C₃-alkyl, - C(=O)-N R⁶R⁶, -C₁-C₃-alkyl-OH, and
         - -NR⁶R⁶,
         wherein R⁶ is independently selected from
         - -H, -C(=O)-O-tert-butyl, -C₁-C₃-alkyl and - C(=O)-CF₃, particularly from -H and Me,
      R⁵ is selected from
         - -NH-SO₂-C₁-C₃-alkyl, -NH-SO₂-(CH₂)ₛ-OH, and
         - -NH-SO₂-(CH₂)ₛ-R⁷,
            wherein R⁷ is selected from an aryl or a heterocycle, wherein the heterocycle is a five or six-membered heterocycle and wherein the heterocycle is an aliphatic heterocycle or an aromatic heterocycle, and
         - -(CH₂)ₛ-R⁸,
            wherein R⁸ is a four or five membered ring,
         s is 0 to 3,
   - 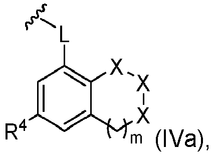 wherein
      L is a linker comprising -(CH₂)ₙ- or -(CH₂)ₙ-Y- and
      wherein Y is selected from -O-, -S-, -SO₂-, -NH- and -C(=O)-NH-, and
      n is 0 to 3,
      n is 0 or 1,
      m is 0 or 1,
      X is independently selected from CH, NR³, SO₂ and O, wherein
      R³ is selected from -H and -C₁-C₃-alkyl-NH₂,
R⁴ is defined as above.

In certain embodiments, L of formula (I) is a linker comprising -(CH₂)ₙ- or -(CH₂)ₙ-Y-, and L of formula (II) is a linker comprising -(CH₂)ₙ- or -Y-(CH₂)ₙ-, wherein Y is selected from -O-, -S-, - SO₂-, -NH- and -C(=O)-NH-, wherein n is 0 or 1, particularly 1.

In certain embodiments L is a linker comprising -(CH₂)ₙ- or -(CH₂)ₙ-Y-, wherein Y is selected from -O-, -S-, -SO₂-, -NH- and -C(=O)-NH-, wherein n is 0 or 1, particularly 1.

In certain embodiments, L is a linker comprising -(CH₂)ₙ-.

In certain embodiments, R² is a moiety of formula (Illa) or (IVa).

In certain embodiments, the compound of formula (Illa) of R² is 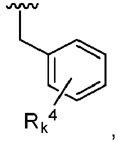 wherein
k is 0 to 3, particularly 0 to 2, more particularly 1 or 2
R⁴ is selected from
   - -Cl, -Br, -CH₂F, -CHF₂, -CF₃, and
   - -C(=O)O-C₁-C₃-alkyl, -C(=O)OH, -C₁-C₃-alkyl, -C(=O)-NR⁶R⁶, -C₁-C₃-alkyl-OH, and
   - -NR⁶R⁶,
      wherein R⁶ is independently selected from
      -H, -C(=O)-O-tert-butyl, -C₁-C₃-alkyl and -C(=O)-CF₃, particularly from -H and Me.

In certain embodiments, R⁴ is selected from
- -Cl, -Br, -CH₂F, -CHF₂, -CF₃,
- -OMe, -C(=O)OMe, -C(=O)OH, -CH₂OH, - NH₂, -C(=O)-NH-Me and -C(=O)-NH₂.

In certain embodiments, R⁴ is selected from
- -Cl, -Br, -CH₂F, -CHF₂, -CF₃,
- -OMe, -CH₂OH, - NH₂, -C(=O)-NH-Me and -C(=O)-NH₂.

In certain embodiments, R⁴ is selected from
-Cl, -OMe, -C(=O)OH, -C(=O)OMe, -C(=O)NHMe and -C(=O)NH₂.

In certain embodiments, R⁴ is selected from
-Cl, -OMe, -C(=O)NHMe and -C(=O)NH₂.

In certain embodiments, R⁴ is selected from
-Cl, -OMe, -C(=O)OH and -C(=O)OMe.

In certain embodiments, R⁴ is -Cl or -C(=O)OMe.

In certain embodiments, R⁴ is -OMe and -Cl.

In certain embodiments, one R⁴ is in meta position to L.

In certain embodiments, the compound of formula (Illa) of R² is 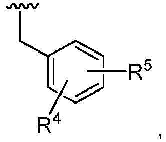 wherein
R⁵ is selected from
   - -NH-SO₂-C₁-C₃-alkyl, -NH-SO₂-(CH₂)ₛ-OH, particularly -NH-SO₂-Me or -NH-SO₂-(CH₂)₂-OH, and
   - -NH-SO₂-(CH₂)ₛ-R⁷, wherein R⁷ is selected from an aryl or a heterocycle, wherein the heterocycle is a five or six-membered heterocycle and wherein the heterocycle is an aliphatic heterocycle or an aromatic heterocycle, and
   - -(CH₂)ₛ-R⁸, wherein R⁸ is a four or five membered ring,
   s is 0 to 3,
R⁴ being as defined above.

In certain embodiments, R⁷ is selected from any one of the moieties

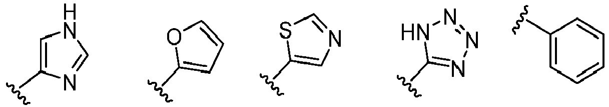

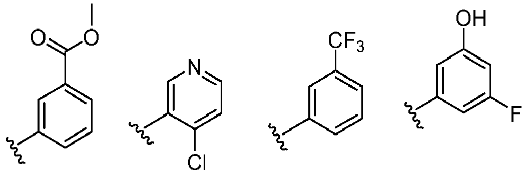

In certain embodiments, R⁸ is selected from any one of the moieties 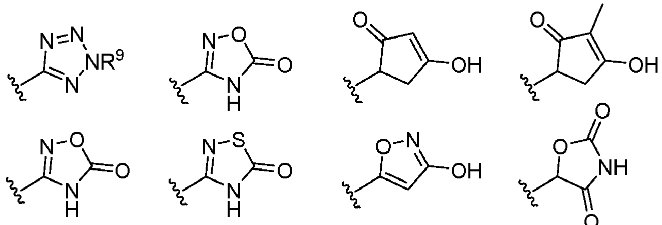 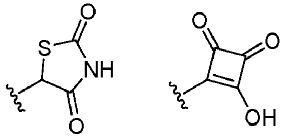 wherein R⁹ is selected from -H or -C₁-C₆-alkyl.

In certain embodiments, R⁸ is selected from any one of the moieties 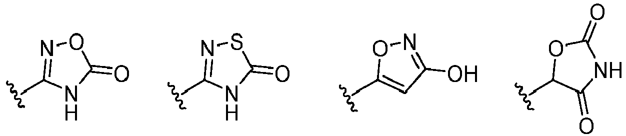 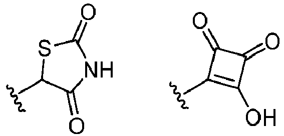 wherein R⁹ is selected from -H or -C₁-C₆-alkyl.

In certain embodiments, R⁸ is selected from any one of the moieties 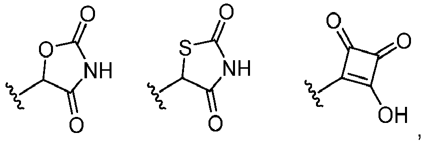 wherein R⁹ is selected from -H or -C₁-C₆-alkyl.

In certain embodiments, R⁵ is selected from any one of the moieties 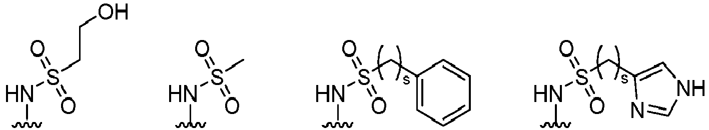 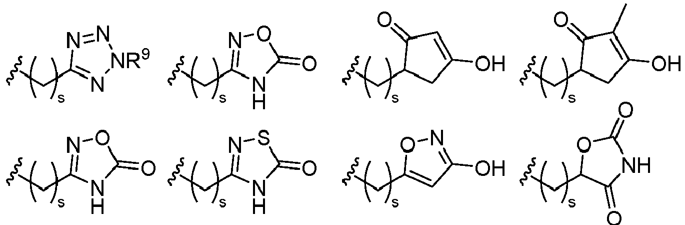 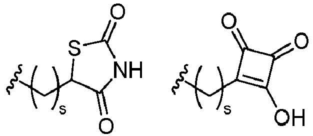 wherein R⁹ is selected from -H or -C₁-C₆-alkyl and wherein s is 0 to 3.

In certain embodiments, R⁵ is selected from any one of the moieties 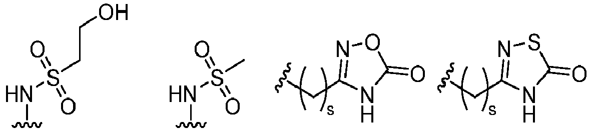 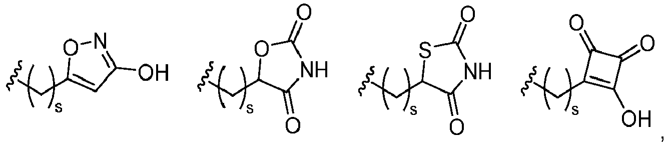 wherein R⁹ is selected from -H or -C₁-C₆-alkyl and wherein s is 0 to 3.

In certain embodiments, R⁵ is selected from any one of the moieties 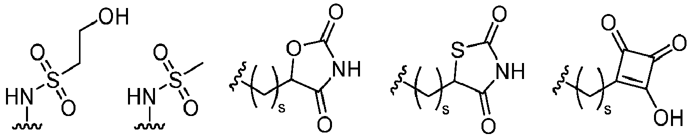 wherein R⁹ is selected from -H or -C₁-C₆-alkyl and wherein s is 0 to 3.

In certain embodiments, one of X is SO₂.

In certain embodiments, at least one of X is NH.

In certain embodiments, the compound of formula (lVa) of R² is selected from any one of the moieties 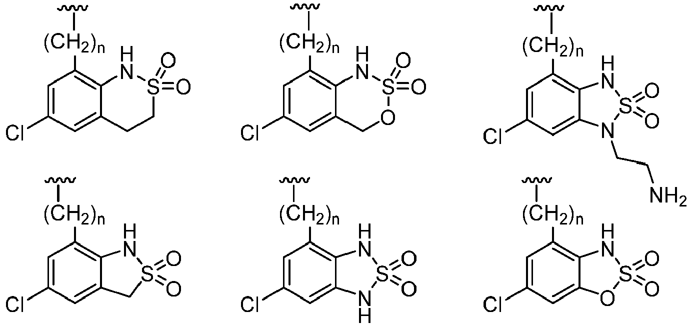 wherein n is 0 or 1.

In certain embodiments, the compound is selected from a compound of formula (FE150), (ZA347), (ZA349), (ZA400) or (ZA364)

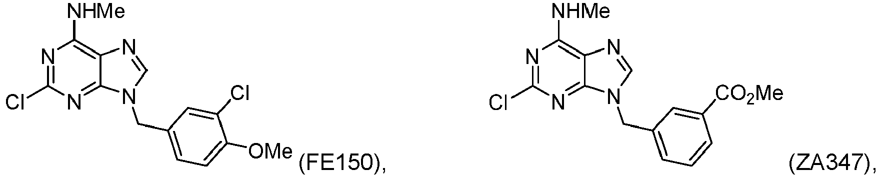

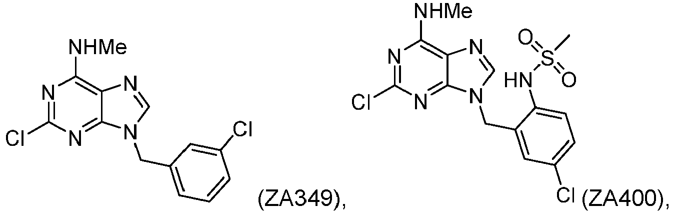

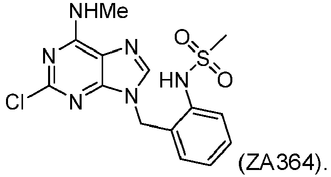

A second aspect of the invention relates to a compound selected from any one of (FE150), (ZA347), (ZA349), (ZA400), (ZA364), (ZA311) and (ZA337) for use as a medicament

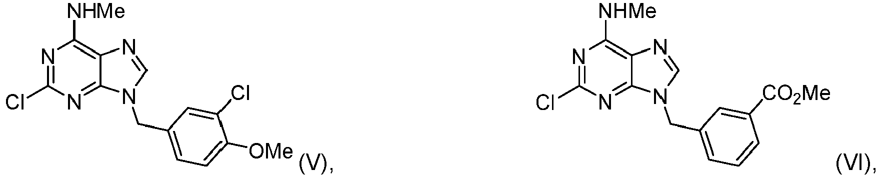

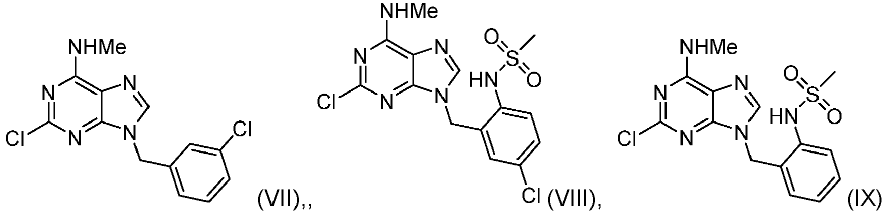

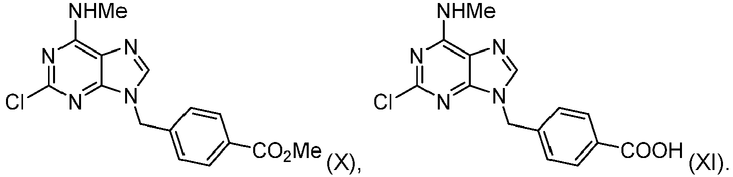

A third aspect of the invention relates to a compound according to the first and second aspect of the invention for use in the treatment of a disease, wherein the disease is cancer.

In certain embodiments, the cancer is selected from renal cancer, breast cancer, acute myeloid leukemia, hepatocellular carcinoma, and lung adenocarcinoma.

### Medical treatment

Similarly, within the scope of the present invention is a method or treating cancer in a patient in need thereof, comprising administering to the patient a compound according to the above description.

### Pharmaceutical Compositions, Administration/Dosage Forms and Salts

According to one aspect of the compound according to the invention, the compound according to the invention is provided as a pharmaceutical composition, pharmaceutical administration form, or pharmaceutical dosage form, said pharmaceutical composition, pharmaceutical administration form, or pharmaceutical dosage form comprising at least one of the compounds of the present invention or a pharmaceutically acceptable salt thereof and at least one pharmaceutically acceptable carrier, diluent or excipient.

The skilled person is aware that any specifically mentioned drug compound mentioned herein may be present as a pharmaceutically acceptable salt of said drug. Pharmaceutically acceptable salts comprise the ionized drug and an oppositely charged counterion. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.

In certain embodiments of the invention, the compound of the present invention is typically formulated into pharmaceutical dosage forms to provide an easily controllable dosage of the drug and to give the patient an elegant and easily handleable product.

Similarly, a dosage form for the prevention or treatment of cancer is provided, comprising a non-agonist ligand or antisense molecule according to any of the above aspects or embodiments of the invention.

The invention further encompasses a pharmaceutical composition comprising a compound of the present invention, or a pharmaceutically acceptable salt thereof, and a pharmaceutically acceptable carrier. In further embodiments, the composition comprises at least two pharmaceutically acceptable carriers, such as those described herein.

Certain embodiments of the invention relate to a dosage form for enteral administration, such as nasal, buccal, rectal, transdermal or oral administration, or as an inhalation form or suppository. In addition, the pharmaceutical compositions of the present invention can be made up in a solid form (including without limitation capsules, tablets, pills, granules, powders or suppositories), or in a liquid form (including without limitation solutions, suspensions or emulsions).

Certain embodiments of the invention relate to a dosage form for parenteral administration, such as subcutaneous, intravenous, intrahepatic or intramuscular injection forms. Optionally, a pharmaceutically acceptable carrier and/or excipient may be present.

The dosage regimen for the compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the compounds of the invention may be administered in a single daily dose, or the total daily dosage may be administered in divided doses of two, three, or four times daily.

In certain embodiments, the pharmaceutical composition or combination of the present invention can be in unit dosage of about 1-1000 mg of active ingredient(s) for a subject of about 50-70 kg. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

### Method of Manufacture and Method of Treatment according to the invention

The invention further encompasses, as an additional aspect, the use of a compound as identified herein, or its pharmaceutically acceptable salt, as specified in detail above, for use in a method of manufacture of a medicament for the treatment or prevention of cancer.

Similarly, the invention encompasses methods of treatment of a patient having been diagnosed with a disease associated with cancer. This method entails administering to the patient an effective amount of compound as identified herein, or its pharmaceutically acceptable salt, as specified in detail herein.

The invention is further illustrated by the following examples and figures, from which further embodiments and advantages can be drawn. These examples are meant to illustrate the invention but not to limit its scope.

### Examples

### Biochemical assays

### Example 1: TR-FRET

The IC₅₀ values were obtained through an HTRF-based assay. The assay evaluates the binding interaction of the YTHDC1 YTH domain (amino acids 345-509) and a methylated RNA (sequence: 5'-Biotin-AAGAACCGG(m⁶A)CUAAGCU-30). The YTH domain of YTHDC1 is expressed as a GST-fusion protein that is recognized by an anti-GST antibody labelled with Eu³⁺, acting as the Förster resonance energy transfer (FRET) donor. The biotinylated RNA is bound by Streptavidin conjugated to XL665, the FRET acceptor. The binding of the methylated RNA to the YTH binding site leads to the formation of a four-member complex constituted by the GST-tagged YTH domain of YTHDC1, anti-GST Eu³⁺-labelled antibody, biotinylated RNA, and Streptavidin conjugated to XL665. This complex formation subsequently leads to the proximity between the FRET donor and acceptor, resulting in a signal emission. Suppose the tested compound can compete with the methylated RNA for the occupation of the YTH active site, the emitted signal decreases. The assay mix includes 25 nM YTHDC1 YTH domain-GST fusion protein, 15 nM biotinylated RNA (Dharmacon), 0.8 nM anti-GST Eu³⁺-conjugated antibody (Cisbio, 61GSTKLB), 1.875 nM XL665-conjugated streptavidin (Cisbio, 610SAXLB), and the compound of interest. The compound concentration in the final mix strictly depends on the assay's aims. It is fixed at 1 mM to investigate the residual signal or set as an array of 1 mM 2-fold dilutions to determine the IC₅₀ values. The assay's components are diluted in a buffer composed of 50 mM HEPES pH 7.5, 150 mM NaCl, 100 mM kF, and 0.1% BSA. The reagents mix is incubated for 3 h at 24 C, transferred into a white, low volume 384-well plate (Corning, 4513), and measured using an Infinite M1000 plate reader (Tecan). Eu³⁺ is excited at a wavelength of 317 nm, and fluorescence emissions are measured at 620 and 665 nm, with an excitation/emission lag time of 60 µs. The IC₅₀ values were obtained by analyzing the dose-response data.

### Example 2: TSA assay

Protein sample was buffered in 25 mM HEPES pH 7.2, 150 mM NaCl and assayed in a 96-well plate at a final concentration of 2 µM in 20 µl volume. Fluorescent dye was added as a fluorescence probe at a dilution of 1:1000. Compound concentrations tested were 12.5 µM, 25 µM, 50 µM, 100 µM and 200 µM. The temperature was raised with a step of 0.5 °C starting from 20 °C to 80 °C and fluorescence readings were taken at each interval. The reported values (ΔTm) are calculated as the difference between the transition midpoints of an individual sample and the average of the reference wells (containing the protein and the DMSO only) in the same plate. The DMSO concentration was kept at 1% (v/v).

### Example 3: Cytotoxicity

Cells were seeded in white clear-bottom 96-well plates at a density of 6 × 10³ cells/well in 50 µL of the complete RPMI medium and treated with 50 µL of increasing concentrations of the indicated compounds dissolved in DMSO (final concentration of compounds 1.25 - 160 µM) or DMSO only (0.5 % (v/v)) as a negative control and incubated for 72 h at 37°C with 5 % CO₂. Cell viability was determined using a CellTiter-Glo luminescent cell viability assay (Promega) based on the detection of ATP according to manufacturer's instructions. 100 µL of the reagent was added to each well and incubated for 10 min at room temperature on an orbital shaker. The luminescence was recorded using a Tecan Infinite 3046 M1000 microplate reader from the top. Background luminescence value was obtained from wells containing the CellTiter-Glo reagent and medium without cells. Cell viability curves were plotted in GraphPad Prism 9 and fitted with nonlinear regression, from which GI₅₀ values were determined.

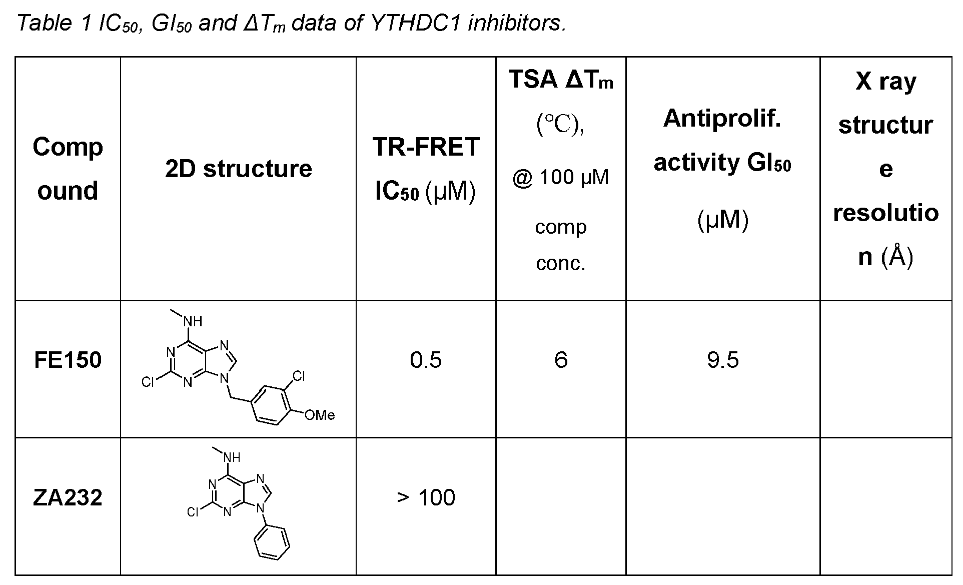

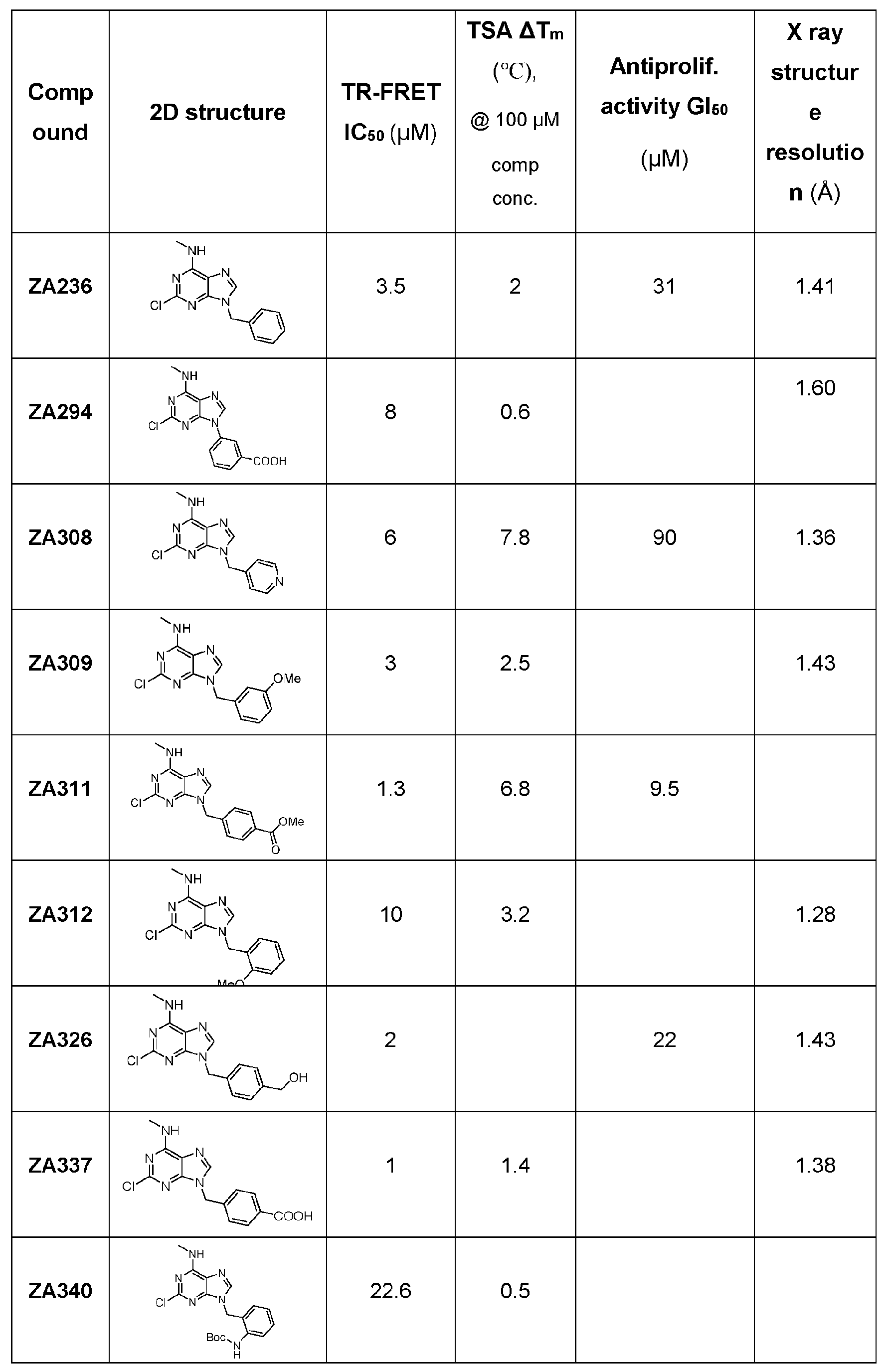

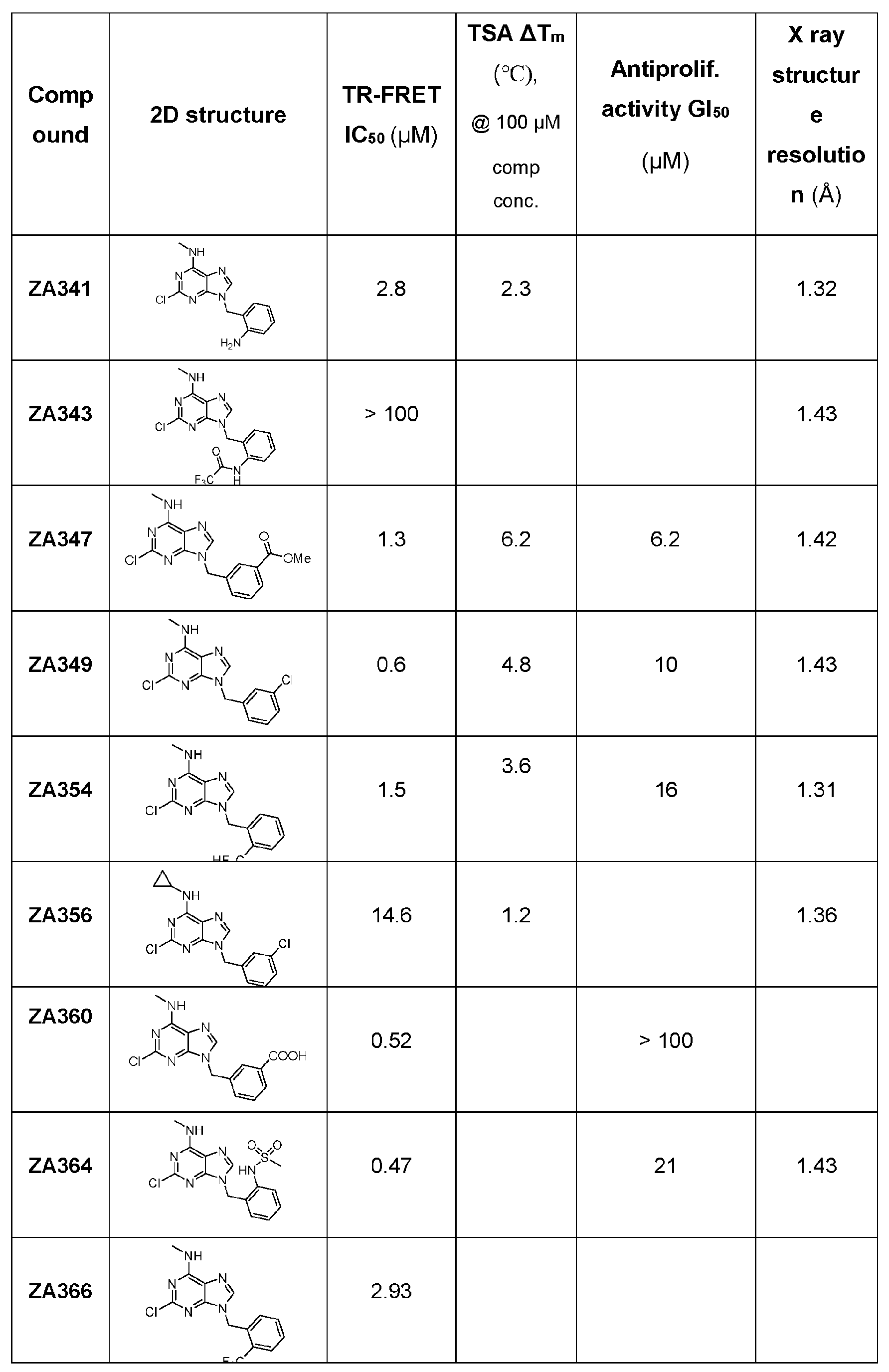

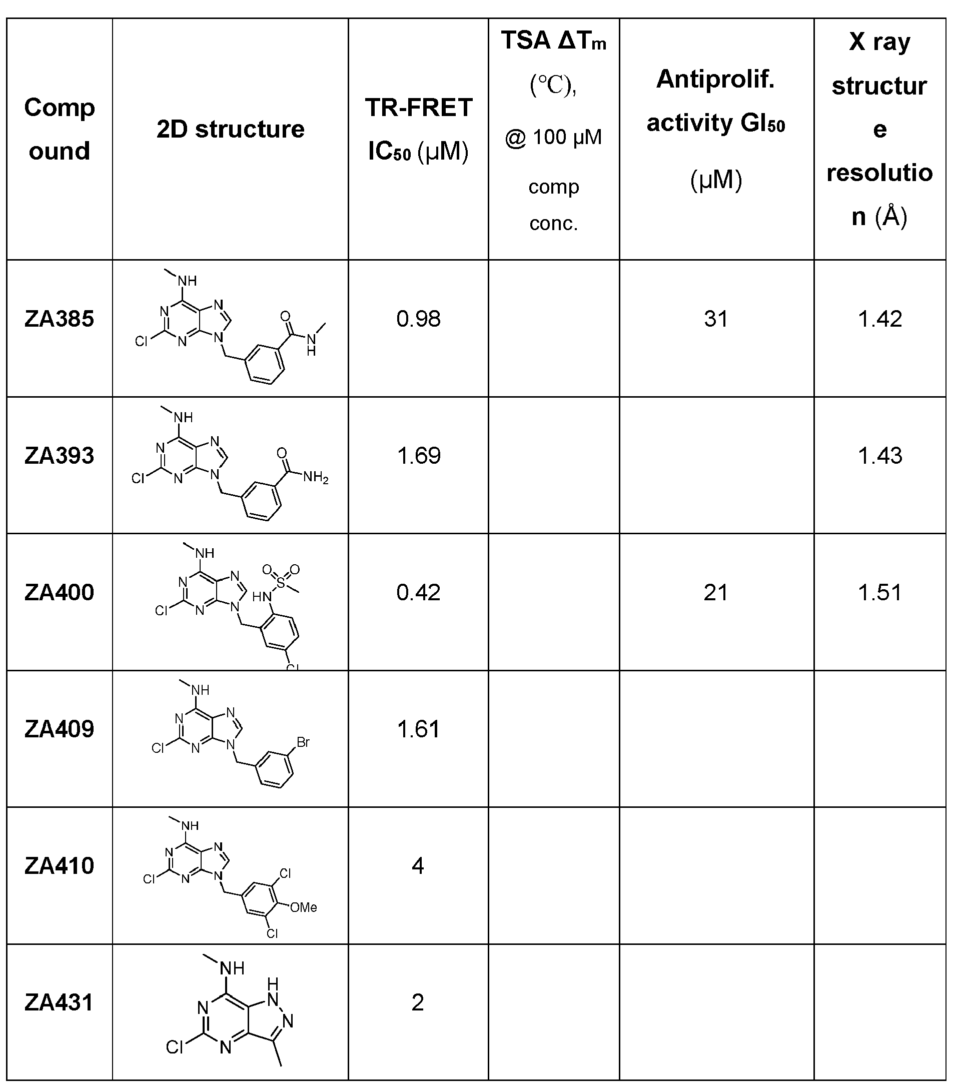

The compounds following formula (I) FE150, ZA311, ZA347 and ZA349 display excellent IC₅₀ values of lower than 1 µM and GI₅₀ values of 10 µM or less. Additionally these compounds show outstanding protein stabilization with temperature shifts between 3.6 to 7.8 °C compared to the unbound protein.

The compounds ZA364 and ZA400 display great IC₅₀ values of 0.47 and 0.42 µM but slightly higher GI₅₀ values of 21 and 31 µM.

The inhibitory effect of compounds ZA236, ZA309, ZA326, ZA341, ZA354 and ZA385 is slightly weaker than of compounds FE150, ZA311, ZA347 and ZA349, with IC₅₀ values of 1.5 to 3.5 µM.

The compound ZA431 following formula (II) shows an inhibitory effect of 2 µM.

### Chemistry

All reagents were purchased from commercial suppliers and used as received. Reactions run at elevated temperature were carried out in the oil bath. All reactions were monitored by thin-layer chromatography (Aluminium plates coated with silica gel 60 F₂₅₄). Flash column chromatography was carried out over silica gel (0.040-0.063 mm). ¹H and ¹³C {¹H} NMR spectra were recorded on AV2 400 MHz and AV600 Bruker spectrometers (400 MHz, 101 MHz and 600 MHz, 150 MHz, respectively) in DMSO or CDCl₃ Chemical shifts are given in ppm and their calibration was performed to the residual ¹H and ¹³C signals of the deuterated solvents. Multiplicities are abbreviated as follows: singlet (s), doublet (d) multiplet (m), and broad signal (bs). The purity was acquired by Liquid chromatography high resolution electrospray ionization mass spectrometry (LC-HR- ESI-MS): Acquity UPLC (Waters, Milford, USA) connected to an Acquity eλ diode array detector and a Synapt G2 HR-ESI-QTOF-MS (Waters, Milford, USA); injection of 1 µL sample (c = ca. 10-100 µg mL-1 in the indicated solvent); Acquity BEH C18 HPLC column (1.7 µm particle size, 2 × 50 mm, Waters) kept at 30 °C;* elution at a flow rate of 400 µL min-1 with A: H₂O + 1% HCO₂H and B: CH₃CN + 0.1% HCO₂H, linear gradient from 5-98% B within 5 min, then isocratic for 1 min;* UV spectra recorded from 200-600 nm at 1.2 nm resolution and 20 points s-1; ESI: positive ionization mode, capillary voltage 3.0 kV, sampling cone 40V, extraction cone 4V, N₂ cone gas 4 L h-1, N₂ desolvation gas 800 L min-1, source temperature 120°C; mass analyzer in resolution mode: mass range 100-2'000 m/z with a scan rate of 1 Hz; mass calibration to <2 ppm within 50-2'500 m/z with a 5mM aq. soln. of HCO₂Na, lockmasses: m/z 195.0882 (caffein, 0.7 ng mL-1) and 556.2771(Leucine-enkephalin, 2 ng mL-1).

### Example 4: General procedure for 2,5-dichloropurine alkylation

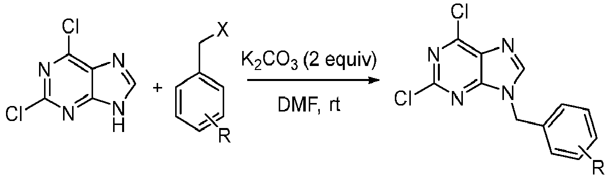

2,6-dichloropurine (1 equiv) was dissolved in DMF (0.5 M) and K₂CO₃ (2 equiv) was added. To a stirred mixture was added alkylhalide (1 equiv). The resulting reaction mixture was stirred at rt until a reaction completion (Monitored by TLC). The reaction mixture was quenched by an addition of water and extracted into EtOAc. Combined organic layers were washed by 10 % aq. sol. of LiCI. Then dried over MgSO₄, filtrated and evaporated.

### Example 5: General procedure for nucleophilic aromatic substitution (SₙAr) with MeNH₂

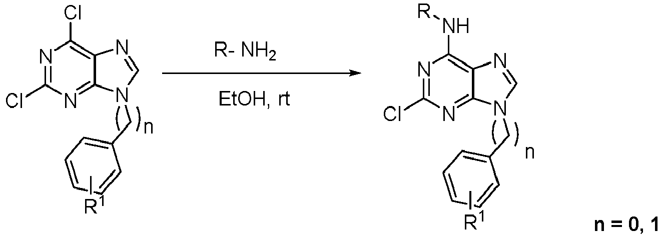

9-alkyl-2,6-dichloropurine (1 mmol) was suspended in EtOH (0.5 M) and 33 % MeNH₂ in EtOH (2 mL) and stirred at rt. After the reaction completion (TLC) the volatiles were removed *in vacuo.* The crude product was purified using flash column chromatography.

### N-(2-((2-chloro-6-(methylamino)-9H-purin-9-yl)methyl)phenyl)methanesulfonamide (ZA364)

To a solution of tert-butyl (2-((2,6-dichloro-9H-purin-9-yl)methyl)phenyl)carbamate (0.067 g, 0.227 mmol) in DCM (2.2 mL) was added pyridine (0.024 mL, 0.295 mmol). The solution was cooled down to 0 °C and MsCI (0.021 mL, 0.274 mmol) was added dropwise after 30 min. After the reaction completion (TLC), the volatiles were removed *in vacuo.* The crude product was subsequently used in the next step without further purification. The final product was prepared following general method for SₙAr. The crude product was purified using flash column chromatography (SiO₂; EtOAc/MeOH = 4 : 1 → 4 : 0) and obtained as a white solid (0.025 g, 30 % after two steps). 1H NMR (400 MHz, DMSO) δ 9.50 (s, 1H), 8.31- 8.25 (m, 1H), 8.18 (s, 1H), 7.40 (dd, *J* = 7.9, 1.5 Hz, 1H), 7.35 (td, *J* = 7.5, 1.5 Hz, 1H), 7.22 (td, *J* = 7.4, 1.5 Hz, 1H), 6.87 (dd, *J* = 7.8, 1.5 Hz, 1H), 5.45 (s, 2H), 3.09 (s, 3H), 2.92 (d, *J* = 4.6 Hz, 2H); 13C NMR (101 MHz, DMSO) δ 155.6, 153.3, 149.6, 141.4, 134.6, 133.3, 128.7, 128.0, 127.1, 126.9, 118.2, 42.9, 27.2. LRMS (ESI) m/z: [M + H]+ calcd for C12H12N5; 367.073 found, 367.075.

### N-(4-chloro-2-((2-chloro-6-(methylamino)-9H-purin-9-yl)methyl)phenyl) methanesulfonamide (ZA400)

To a solution of N-(4-chloro-2-(hydroxymethyl)phenyl)methanesulfonamide (0.747 g, 3.17 mmol) in DCM (6.3 mL) was added SOCl₂ (0.342 mL, 4.75 mmol) dropwise. The reaction mixture was stirred at rt for 90 min. The volatiles were removed *in vacuo* and the crude product was used dissolved in DMF (6.2 mL). To a stirred solution 2,6-dichloropurine (0.599 g, 3.17 mmol) and K₂CO₃ (0.649 g, 4.71 mmol) was added. The reaction was quenched by aq. sol. of NH₄Cl after 5 hours. The reaction mixture was extracted into EtOAc (3 × 12 mL) and combined organic layers were dried over MgSO₄, filtrated and evaporated. The residue was dissolved in EtOH (4 mL) and 33 % MeNH₂ in EtOH (1 mL) was added. The reaction mixture was stirred at rt for 1 hour. After the reaction completion, the volatiles were removed *in vacuo.* Crude final compound was purified using flash column chromatography (SiO2; EtOAc/Hept = 4 : 1 → 6 : 1) and obtained as a white solid (0.074 g, 6 % after three steps). 1H NMR (400 MHz, DMSO) δ 9.61 (s, 1H), 8.32 - 8.27 (m, 1H), 8.21 (s, 1H), 7.45 - 7.40 (m, 2H), 5.43 (s, 2H), 3.10 (s, 3H), 2.93 (d, *J* = 4.6 Hz, 3H); ¹³C NMR (101 MHz, DMSO) δ 155.6, 153.3, 149.6, 141.4, 134.6, 133.3, 128.7, 128.0, 127.1, 126.9, 118.2, 42.9, 27.2. LRMS (ESI) m/z: [M + H]+ calcd for C14H15Cl2N6O2S; 401.035 found, 401.035.

N-(4-chloro-2-(hydroxymethyl)phenyl)methanesulfonamide was prepared according to 4).

### 2-chloro-9-(3-chloro-4-methoxybenzyl)-N-methyl-9H-purin-6-amine (FE150)

The compound was prepared following general method for SₙAr starting from corresponding 9-alkyl-2,6-dichloro-9H-purine (0.134 g, 0.397 mmol). The crude product was purified using flash column chromatography (SiO₂; EtOAc/Hept = 2 : 3 → 1 : 4) and obtained as a white solid (0.08 g, 59 %). %). 1H NMR (400 MHz, DMSO) δ 8.24 (s, 1H), 8.26 - 8.18 (bs, 1H), 7.44 (d, *J* = 2.2 Hz, 1H), 7.25 (dd, *J* = 8.5, 2.2 Hz, 1H), 7.12 (d, *J* = 8.5 Hz, 1H), 5.26 (s, 2H), 3.82 (s, 3H), 2.91 (d, *J* = 4.6 Hz, 3H); 13C NMR (101 MHz, DMSO) δ 155.5, 154.2, 153.4, 149.3, 141.0, 129.8, 129.3, 127.9, 121.0, 118.3, 113.0, 56.1, 45.3, 27.2. LRMS (ESI) m/z: [M + H]+ calcd for C14H14Cl2N5O; 338.057 found, 338.057.

### Methyl 3-((2-chloro-6-(methylamino)-9H-purin-9-yl)methyl)benzoate (ZA347)

The compound was prepared following general method for SₙAr starting from corresponding 9-alkyl-2,6-dichloro-9H-purine (0.465 g, 1.38 mmol). The crude product was purified using flash column chromatography (SiO₂; EtOAc/Hept = 3:1) and obtained as a white solid (0.374g, 82 %). ¹H NMR (400 MHz, DMSO) δ 8.28 (s, 1H), 8.29 - 8.21 (m, 1H), 7.90 - 7.87 (m, 2H), 7.56 - 7.49 (m, 2H), 5.43 (s, 2H), 3.83 (s, 3H), 2.92 (d, *J* = 4.6 Hz, 1H); 13C NMR (101 MHz, DMSO) δ 165.9, 155.6, 153.5, 149.5, 141.2, 137.5, 132.3, 130.1, 129.4, 128.6, 128.0, 118.3, 52.3, 45.9, 27.2. LRMS (ESI) m/z: [M + H]+ calcd for C15H15ClN5O2; 332.091 found, 332.091.

### 2-chloro-9-(3-chlorobenzyl)-N-methyl-9H-purin-6-amine (ZA349)

The compound was prepared following general method for SₙAr starting from corresponding 9-alkyl-2,6-dichloro-9H-purine (0.272 g, 0.867 mmol). The crude product was purified using flash column chromatography (SiO₂; EtOAc/Hept = 3: 1) and obtained as a white solid (0.191 g, 71 %). ¹H NMR (400 MHz, DMSO) δ 8.26 (s, 1H), 8.28 - 8.21 (bs, 1H), 7.40 - 7.35 (m, 3H), 7.23 - 7.18 (m, 1H), 5.35 (s, 2H), 2.92 (d, *J* = 4.7 Hz, 3H); 13C NMR (101 MHz, DMSO) δ 155.6, 153.5, 149.4, 141.2, 139.2, 133.3, 130.8, 127.9, 127.4, 126.1, 118.3, 45.7, 27.2. LRMS (ESI) m/z: [M + H]+ calcd for C13H12Cl2N5; 308.045 found, 308.046.

### Methyl 4-((2-chloro-6-(methylamino)-9H-purin-9-yl)methyl)benzoate (ZA311)

The compound was prepared following general method for SₙAr starting from corresponding 9-alkyl-2,6-dichloro-9H-purine (0.052 g, 0.154 mmol). The crude product was purified using flash column chromatography (SiOz; EtOAc/Hept = 2: 1) and obtained as a white solid (0.038 g, 74 %). ¹H NMR (400 MHz, DMSO) δ 8.26 (s, 2H), 7.93 (d, 2H), 7.35 (d, 2H), 5.45 (s, 2H), 3.83 (s, 3H), 2.92 (bs, 3H); 13C NMR (101 MHz, DMSO) δ 165.9, 155.6, 153.5, 149.5, 142.4, 141.3, 129.6, 129.0, 127.4, 118.3, 52.2, 45.9, 27.2. HRMS (ESI) m/z: [M + H]+ calcd for C15H15CIN5O2; 332.091 found, 332.091.

### 3-((2-chloro-6-(methylamino)-9H-purin-9-yl)methyl)benzoic acid (ZA337)

The corresponding methyl ester (0.2 g, 0.602 mmol) was dissolved in dioxane (6 mL) followed by the addition of 38 % HCl (4 mL). The reaction mixture was refluxed for 5 hours. The reaction mixture was cooled to 0 °C and left for 4 hours at this temperature. The white precipitate was filtered off and dried on air. The carboxylic acid was isolated as a white solid (164 mg, 85 %). 1H NMR (400 MHz, DMSO) δ 8.52 (s, 1H), 8.48 - 8.38 (bs, 1H), 7.92 - 7.88 (m, 2H), 7.35 (d, *J* = 8.1 Hz, 2H), 5.47 (s, 2H), 2.93 (s, 3H); 13C NMR (101 MHz, DMSO) δ 13C NMR (101 MHz, DMSO) 13C NMR (101 MHz, DMSO) δ 167.0, 155.1, 154.0, 149.3, 141.2, 141.0, 130.4, 129.8, 127.5, 116.8, 46.4, 27.3. LRMS (ESI) m/z: [M + H]+ calcd for C14H13CIN5O2 318.075 found, 318.075.

### 5-chloro-N,3-dimethyl-1H-pyrazolo[4,3-d]pyrimidin-7-amine (ZA431)

5,7-dichloro-3-methyl-1H-pyrazolo[4,3-d]pyrimidine (0.101 g, 0.499 mmol) was suspended in EtOH (0.5 mL). To the suspension was added 33% MeNH2 in EtOH (0.2 mL) and the reaction was stirred for 1 hour at room temperature. After the reaction completion (TLC) the volatiles were removed in vacuo. The crude product was purified using flash column chromatography (SiO2; EtOAc/MeOH = 10 : 1) and obtained as a yellowish solid (0.018 g, 18 %). 1H NMR (400 MHz, MeOD - d4) δ 3.12 (s, 3H), 2.49 (s, 3H); 13C NMR (101 MHz, DMSO) 27.8, 9.4; δ LRMS (ESI) m/z: [M + H]+ calcd for C7H9CIN5; 198.6 found, 198.0.

### 5,7-dichloro-3-methyl-1H-pyrazolo[4,3-d]pyrimidine was prepared according to 5).

### Cited prior art documents:

1) Sambrook et al., Molecular Cloning: A Laboratory Manual, 4th ed. (2012) Cold Spring Harbor Laboratory Press.
2) Ausubel et al., Short Protocols in Molecular Biology (2002) 5th Ed, John Wiley & Sons, Inc.
3) L. Lachman et al. The Theory and Practice of Industrial Pharmacy, 4th Ed, 2013 (ISBN 8123922892).
4) Wu, Shuquan; Liu, Changyi; Luo, Guoyong; Jin, Zhichao; Zheng, Pengcheng; Chi, Yonggui Robin[Angewandte Chemie - International Edition, 2019, vol. 58, # 51, p. 18410 - 18413][Angew. Chem., 2019, vol. 131, p. 18581 - 18584,4].
5) Pfizer Limited, WO 2004/96810 A1.

All scientific publications and patent documents cited in the present specification are incorporated by reference herein.

## Claims

1. A compound of formula (I) or (II), particularly of (I) 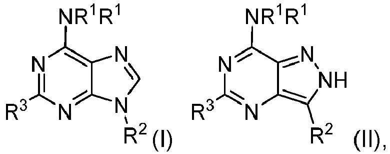 wherein
R¹ is independently selected from -H and -C₁-C₃-alkyl, particularly -H and -Me or cyclopropyl, more particularly -H and -Me, wherein at least one of R¹ is H,
R³ is selected from -Br, -Cl, -CN, -F and -NO₂, particularly -Cl,
R² is selected from -C₁-C₃-alkyl and a moiety of formula (III) or (IV), particularly
R² is a moiety of formula (III) or (IV),
• 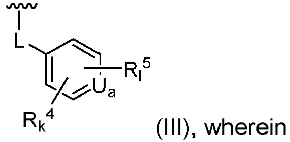
L of formula (I) is a linker comprising -(CH₂)ₙ- or -(CH₂)ₙ-Y- and
L of formula (II) is a linker comprising -(CH₂)ₙ- or -Y-(CH₂)ₙ-, wherein Y is selected from -O-, -S-, -SO₂-, -NH- and -C(=O)-NH-, and
n is 0 to 3, k is 0 to 3, particularly 0 to 2, more particularly 1 or 2, I is 0 or 1,
U is CH or a heteroatom, particularly CH or N, more particularly CH,
a is 0 or 1, particularly 1,
R⁴ is independently selected from
- -Cl, -Br, -CH₂F, -CHF₂, -CF₃ and
- -C(=O)O-C₁-C₃-alkyl, -C(=O)OH, -O-C₁-C₃-alkyl, - C(=O)-NR⁶R⁶, -C₁-C₃-alkyl-OH, and
- -NR⁶R⁶,
wherein R⁶ is independently selected from
- -H, -C(=O)-O-tert-butyl, -C₁-C₃-alkyl, and - C(=O)-CF₃, particularly from -H and Me,
R⁵ is selected from
- -NH-SO₂-C₁-C₃-alkyl, -NH-SO₂-(CH₂)ₛ-OH and
- -NH-SO₂-(CH₂)ₛ-R⁷, wherein R⁷ is selected from an aryl and a heterocycle, wherein the heterocycle is a five or six-membered heterocycle and wherein the heterocycle is an aliphatic heterocycle or an aromatic heterocycle, and
- -(CH₂)ₛ-R⁸, wherein R⁸ is a four or five membered ring,
s is 0 to 3,
or
• 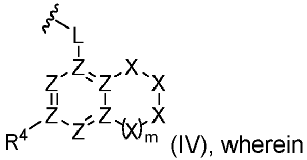
L of formula (I) is a linker comprising -(CH₂)ₙ- or -(CH₂)ₙ-Y- and
L of formula (II) is a linker comprising -(CH₂)ₙ- or -Y-(CH₂)ₙ-, wherein Y is selected from -O-, -S-, -SO₂-, -NH- and -C(=O)-NH-, and
n is 0 to 3, m is 0 or 1,
each X and Z are selected from C, NR³, SO₂ and O, wherein R³ is -H or -C₁-C₃-alkyl-NH₂ and wherein Z is particularly C,
R⁴ is defined as above,
with the exception of

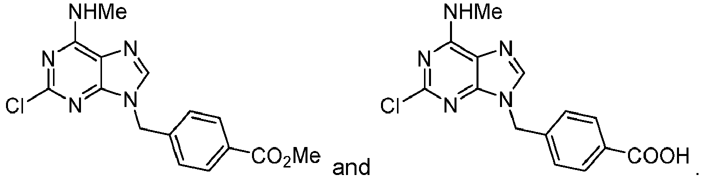

2. The compound according to claim 1 is a compound of formula (Ia) or (IIa), particularly of (la) 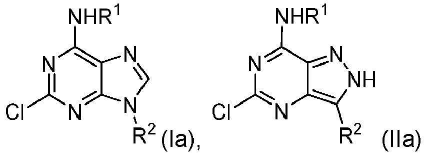 wherein
R¹ is selected from -H and -C₁-C₃-alkyl, particularly -H and -Me or cyclopropyl, more particularly -Me,
R² is selected from -C₁-C₃-alkyl and a moiety of formula (Illa) or (lVa), particularly R² is a moiety of formula (IIIa) or (IVa),
• 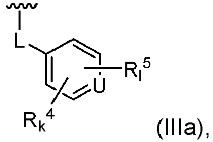 wherein
L of formula (I) is a linker comprising -(CH₂)ₙ- or -(CH₂)ₙ-Y- and
L of formula (II) is a linker comprising -(CH₂)ₙ- or -Y-(CH₂)ₙ-, wherein Y is selected from -O-, -S-, -SO₂-, -NH- and -C(=O)-NH-, and
and n is 0 to 3,
k is 0 to 3, particularly 0 to 2, more particularly 1 or 2,
I is 0 or 1,
U is CH or a heteroatom, particularly CH or N, more particularly CH,
R⁴ is selected from
- -Cl, -Br, -CH₂F, -CHF₂, -CF₃, and
- -C(=O)O-C₁-C₃-alkyl, -C(=O)OH, -O-C₁-C₃-alkyl, - C(=O)-N R⁶R⁶, -C₁-C₃-alkyl-OH, and
- -NR⁶R⁶,
wherein R⁶ is independently selected from
- -H, -C(=O)-O-tert-butyl, -C₁-C₃-alkyl and - C(=O)-CF₃, particularly from -H and Me, 35
R⁵ is selected from
- -NH-SO₂-C₁-C₃-alkyl, -NH-SO₂-(CH₂)ₛ-OH, and
- -NH-SO₂-(CH₂)ₛ-R⁷,
wherein R⁷ is selected from an aryl or a heterocycle, wherein the heterocycle is a five or six-membered heterocycle and wherein the heterocycle is an aliphatic heterocycle or an aromatic heterocycle, and
- -(CH₂)ₛ-R⁸,
wherein R⁸ is a four or five membered ring,
s is 0 to 3,
• 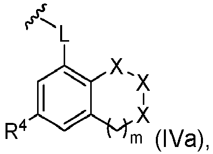 wherein
L of formula (I) is a linker comprising -(CH₂)ₙ- or -(CH₂)ₙ-Y- and
L of formula (II) is a linker comprising -(CH₂)ₙ- or -Y-(CH₂)ₙ-, wherein Y is selected from -O-, -S-, -SO₂-, -NH- and -C(=O)-NH-, and
n is 0 to 3,
n is 0 or 1,
m is 0 or 1,
X is independently selected from CH, NR³, SO₂ and O, wherein
R³ is selected from -H and -C₁-C₃-alkyl-NH₂,
R⁴ is defined as above.

3. The compound according to any of the preceding claims, wherein L of formula (I) is a linker comprising -(CH₂)ₙ- or -(CH₂)ₙ-Y-, and L of formula (II) is a linker comprising - (CH₂)ₙ- or -Y-(CH₂)ₙ-, wherein Y is selected from -O-, -S-, -SO₂-, -NH- and -C(=O)-NH-, wherein n is 0 or 1, particularly 1.

4. The compound according to any of the preceding claims, wherein formula (Illa) of R² is 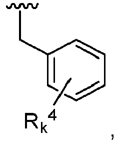 wherein
k is 0 to 3, particularly 0 to 2, more particularly 1 or 2
R⁴ is selected from
- -Cl, -Br, -CH₂F, -CHF₂, -CF₃, and
- -C(=O)O-C₁-C₃-alkyl, -C(=O)OH, -C₁-C₃-alkyl, -C(=O)-NR⁶R⁶, -C₁-C₃-alkyl-OH, and
- -NR⁶R⁶,
wherein R⁶ is independently selected from
-H, -C(=O)-O-tert-butyl, -C₁-C₃-alkyl and -C(=O)-CF₃, particularly from -H and Me.

5. The compound according to any of the preceding claims, wherein R⁴ is selected from
- -Cl, -Br, -CH₂F, -CHF₂, -CF₃,
- -OMe, -C(=O)OMe, -C(=O)OH, -CH₂OH, - NH₂, -C(=O)-NH-Me and -C(=O)-NH₂,
particularly from
-Cl, -OMe, -C(=O)OH, -C(=O)OMe, -C(=O)NHMe and -C(=O)NH₂,
more particularly from
-Cl, -OMe, -C(=O)OH and -C(=O)OMe.

6. The compound according to any of the preceding claims, wherein formula (Illa) of R² is 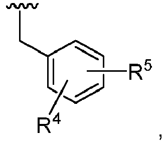 wherein
R⁵ is selected from
- -NH-SO₂-C₁-C₃-alkyl, -NH-SO₂-(CH₂)ₛ-OH, particularly -NH-SO₂-Me or -NH-SO₂-(CH₂)₂-OH, and
- -NH-SO₂-(CH₂)ₛ-R⁷, wherein R⁷ is selected from an aryl or a heterocycle, wherein the heterocycle is a five or six-membered heterocycle and wherein the heterocycle is an aliphatic heterocycle or an aromatic heterocycle, and
- -(CH₂)ₛ-R⁸, wherein R⁸ is a four or five membered ring,
s is 0 to 3,
R⁴ being as defined above.

7. The compound according to any of the preceding claims, wherein R⁷ is selected from any one of the moieties

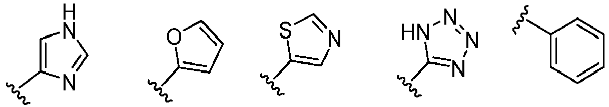

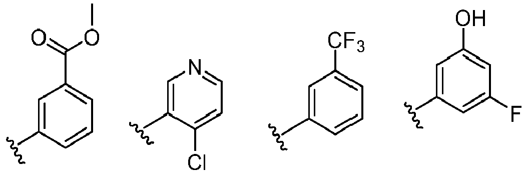

8. The compound according to any of the preceding claims, wherein R⁸ is selected from any one of the moieties 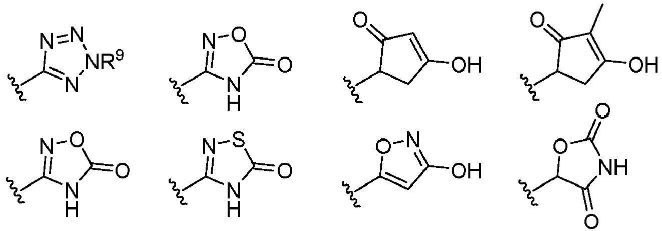 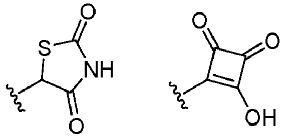 particularly from any one of the moieties 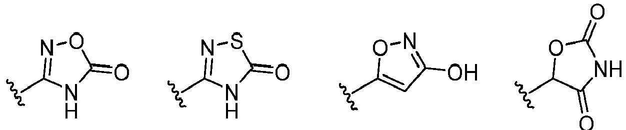 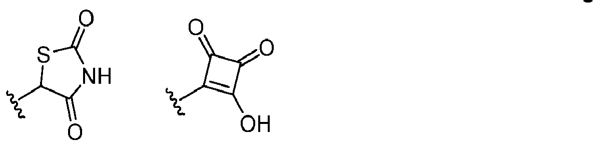 more particularly from any one of the moieties 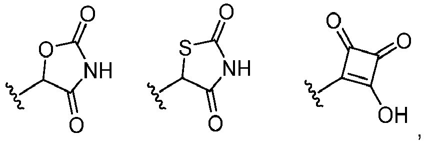 wherein R⁹ is selected from -H or -C₁-C₆-alkyl.

9. The compound according to any of the preceding claims, wherein R⁵ is selected from any one of the moieties 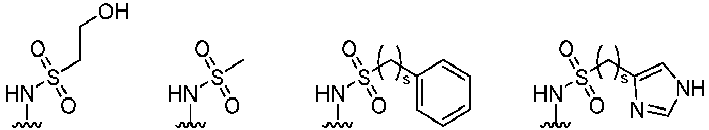 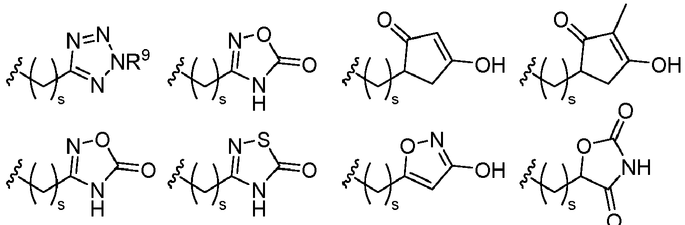 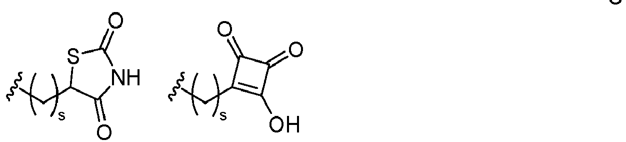 particularly from any one of the moieties 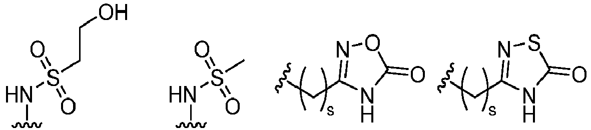 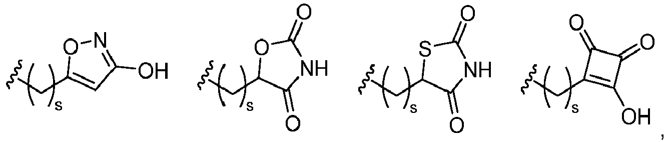 more particularly from any one of the moieties 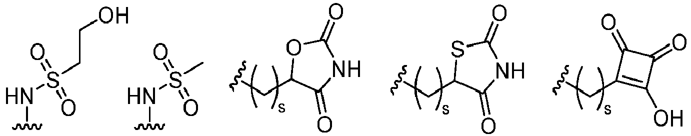 wherein R⁹ is selected from -H or -C₁-C₆-alkyl and wherein s is 0 to 3.

10. The compound according to any of the preceding claims, wherein one of X is SO₂.

11. The compound according to any of the preceding claims, wherein at least one of X is NH.

12. The compound according to any of the preceding claims, wherein formula (lVa) of R² is selected from any one of the moieties 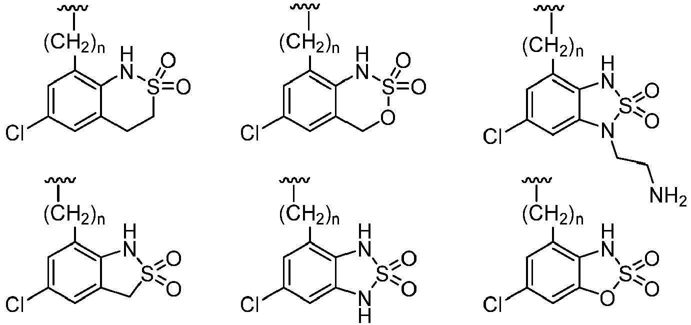 wherein n is 0 or 1.

13. The compound according to any of the preceding claims, wherein the compound is selected from a compound of formula (FE150), (ZA347), (ZA349), (ZA400) or (ZA364)

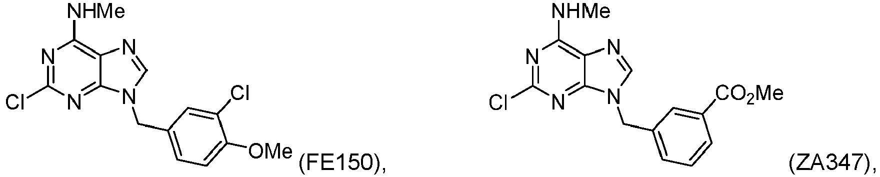

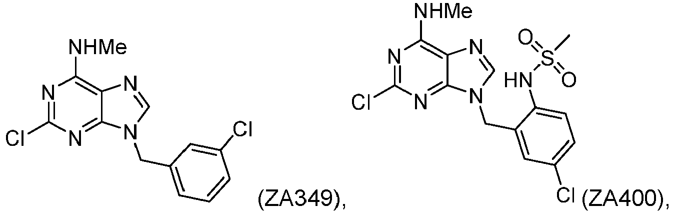

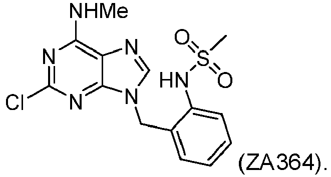

14. A compound according to claims 1 to 13, or a compound of (ZA311) and (ZA3337) for use as a medicament

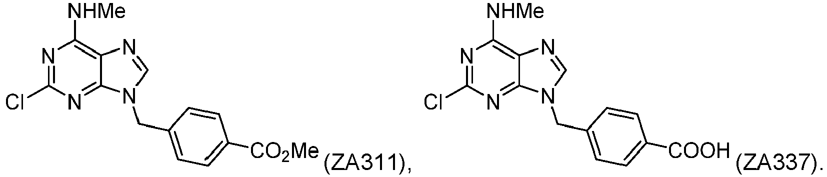

15. A compound according to claims 1 to 13 or a compound of (ZA311) and (ZA3337) 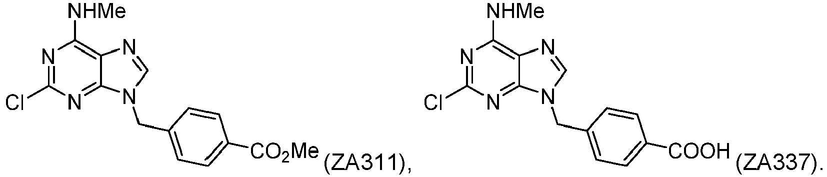 for use in the treatment of a disease, wherein the disease is cancer, in particular the cancer is selected from breast cancer, renal cancer, acute myeloid leukemia, hepatocellular carcinoma, and lung adenocarcinoma.
